# EUROPEAN PATENT SPECIFICATION

(11) **EP 4 132 485 B1**
(45) Date of publication and mention of the grant of the patent: **25.02.2026**
(21) Application number: 21718666.7
(22) Date of filing: 07.04.2021
(51) Int. Cl.: A61K 31/05, A61P 11/00, A61P 31/04, A61P 31/14, A61K 9/00

(54) **PHENOL FOR USE IN THE TREATMENT OF CORONAVIRUS INDUCED DYSPNOEA**
PHENOL ZUR VERWENDUNG BEI DER BEHANDLUNG VON DURCH CORONAVIRUS INDUZIERTER DYSPNOE
PHÉNOL POUR L'UTILISATION DANS LE TRAITEMENT DE LA DYSPNÉE INDUITE PAR VOIE CORONAVIRUS

(30) Priority: 07.04.2020 NL 2025295; 07.04.2020 US 202063006301 P
(43) Date of publication of application: 15.02.2023
(73) Proprietor: LIN, Wen Zhi, 2242 ST Wassenaar (NL)
(72) Inventor: LIN, Wen Zhi, 2242 ST Wassenaar (NL)
(74) Representative: Wittop Koning, Tom Hugo
(86) International application number: PCT/NL2021/050225
(87) International publication number: WO 2021/206549

(56) References cited:
- EP-A1- 0 707 849
- WO-A1-2012/159444
- CN-A- 102 198 091
- YUE-HUA LI: "Hypothesis: Phenol Can Be Antiviral", 10 October 2019 (2019-10-10), pages 1 - 22, XP55821332, Retrieved from the Internet <URL:https://drive.google.com/drive/folders/1CAKmU3E1ys9ijHmg08_QWHDwAoAHwoo2?fbclid=IwAR2ZtoCXRGkoy-BNkC2KkPCkXj-gwLUokZWVTZZJFuEglUQYsRLU4HC0KHM> [retrieved on 20210706]
- ONG E: "'Big pot medicine' and 'divine doctors' a recipe for disaster", 5 March 2020 (2020-03-05), XP055932959, Retrieved from the Internet <URL:https://www.thinkchina.sg/big-pot-medicine-and-divine-doctors-recipe-disaster> [retrieved on 20220620]
- ANON: "Phenol acupoint injection Facebook Post", 27 March 2020 (2020-03-27), XP055898275, Retrieved from the Internet <URL:https://www.facebook.com/pg/Phenol-acupoint-injection%E8%8B%AF%E9%85%9A%E7%A9%B4%E4%BD%8D%E6%B3%A8%E5%B0%84%E7%96%97%E6%B3%95-109324417380461/posts/> [retrieved on 20220307]
- HASAN DJO ET AL: "A novel definition and treatment of hyperinflammation in COVID-19 based on purinergic signalling", PURINERGIC SIGNALLING, SPRINGER VERLAG, DE, vol. 18, no. 1, 10 November 2021 (2021-11-10), pages 13 - 59, XP037697925, ISSN: 1573-9538, [retrieved on 20211110], DOI: 10.1007/S11302-021-09814-6
- ANON: "Phenol Toxicological Overview", 1 February 2016 (2016-02-01), pages 1 - 14, XP055755898, Retrieved from the Internet <URL:https://assets.publishing.service.gov.uk/government/uploads/system/uploads/attachment_data/file/500822/Phenol_PHE_TO_120216.pdf> [retrieved on 20201202]
- YUE-HUA LI: "Hypothesis: Phenol Can Be Antiviral", 10 October 2019 (2019-10-10), pages 1 - 22, XP055821332, Retrieved from the Internet <URL:https://drive.google.com/drive/folders/1CAKmU3E1ys9ijHmg08_QWHDwAoAHwoo2?fbclid=IwAR2ZtoCXRGkoy-BNkC2KkPCkXj-gwLUokZWVTZZJFuEglUQYsRLU4HC0KHM> [retrieved on 20210706]

## Description

The invention relates to the novel use of phenol treatment of microbially induced dyspnoea in a patient. The invention is set out in the appended set of claims.

Phenol was discovered in 1834 by F.F. Runge by extraction from coal tar. It was obtained in pure form by A. Laurent in 1841. In the medical world, phenol has widely been used as antiseptic. Presently, phenol is commonly used in matrixectomy. Also, phenol is used a preservative in injectables such as insulin and vaccines.

As active ingredient, phenol is known for treatment of sore throat. Further, CN102198091 describes the use of phenol as therapeutic drug against some chronical inflammations and herpes zoster, however without providing a mechanism of action of phenol in the therapeutic process. The use of phenol has not been further explored.

EP0707849 describes the use of iodophenolic compounds that can be administered both orally and parenterally for treatment of a plurality of infections and asthma. Yue-Hua Li: Abstractt "Hypothesis: Phenol Can Be Antiviral",, 10 October 2019 (2019-10-10) already teaches the use of parenteral phenol infusions to treat viral bronchitis or pneumonia.

When a body is invaded by external pathological factors, the immune system needs time to build up till the level which can cure the illness again. We all know that after a common cold, e.g. as a result from a viral of bacterial infection, most people naturally get better within a week. The microbe (i.e. the virus or bacterium) does not damage the organs and tissues directly. The damage is caused mostly by changes of the immune system. Microbially induced dyspnoea triggers the activation of the proinflammatory response of the innate immunity causing a massive cytokine storm and tissue damage by strong oxidation. In order to remove the virus from the body, the body's immune system activity increases, and therewith the oxidation activity. Over-oxidation in the body is both cause and consequence of the inflammatory storm. This vicious circle ultimately undermines the recover possibility.

After infection and stress due to illness, the oxidation level of the body is high. The concentration of free radicals increases throughout the body with higher concentration near the infection location and stress affected area such as the airway and lungs, the stress areas being the neck and head. Both infection and stress area may spread further.

As defence against over-oxidation, the body produces catalase, an enzyme that decomposes hydrogen peroxide (H₂O₂), before hydroxyl or superoxide radicals are formed.

It has now surprisingly been found that phenol can be used in the treatment of microbially induced dyspnoea in a patient, in particular in a human patient by parenteral administration. Without the wish to be bound to any explanation it is believed that phenol can be converted in the body into catechol and hydroxyquinone that have antioxidant function. The physiological conversion of phenol is catalysed by glycerol and consumes H₂O₂ as depicted in the reaction scheme below:

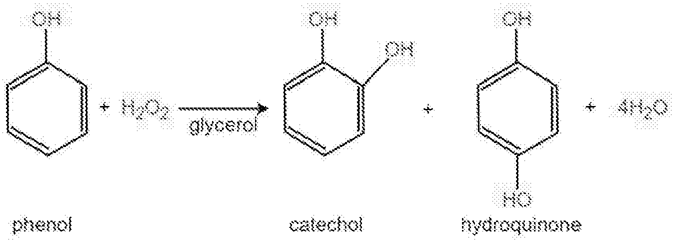

By the above reaction, both phenol and the reaction products phenolic provide antioxidant activity against the inflammatory storm in the infection and more in particular when the dyspnoea is associated with Acute Respiratory Distress Syndrome (ARDS) or pneumonia.

In particular the microbially induced dyspnoea is caused by a viral or bacterial infection that can cause pneumonia. The microbially induced dyspnoea is in particular caused by a virus, chosen from the group, consisting of corona, influenza, Ebola, respiratory syncytial virus (RSV), HIV, Lassa and rhinovirus, as such viral infections, in particular when acute, become life threatening for the patient as a result of dyspnoea.

The Acute Respiratory Distress Syndrome (ARDS) caused by coronavirus disease 2019 (CoViD-19) has now become the world's number 1 challenge. The exponential pattern in the number of severe cases has shown to reach nations' maximum ICU capacities in weeks rather than months after outbreak of the disease irrespective of rigorous population based preventive measures. The CoVid-19 (SARS-CoV-2) virus emerged in December 2019 and turned pandemic. No antiviral medication or effective and safe drug is available yet to treat CoVid-19 patients.

As the provision of vaccines and/or targeted anti-viral medication is cumbersome and depends on the production of a limited number of providers, therapeutic measures that can immediately attenuate the course of CoViD-19-related lung damage potentially are directly needed on a global scale.

It has been found that parenteral administration, relief and quick recovery of the patient from dyspnoea and the underlying infection is observed. The parenteral administration is by subcutaneous injection or intravenous administration.

The patient can be any animal that suffers from microbially induced dyspnoea, in particular mammal, preferably livestock or companion animals. Most preferred patients are human.

It has been found that parenteral administration, i.e., subcutaneous or intravenous administration, is in particular effective when the patient suffers from dyspnoea, in particular induced by microbial infection, such as viral infection, such as corona such as COVID-19, influenza, Ebola, respiratory syncytial virus (RSV), HIV, Lassa and rhinovirus, dengue, chikungunya; bacterial infection, such as meningitis, multiresistant Staphylococcus aureus (MRSA); pneumonia, in particular flue induced pneumonia; acute respiratory distress syndromes (ARDS).

The phenol is preferably in a physiological saline solution, i.e. preferably administered in a composition comprising phenol and physiological saline. Physiological saline is a saline solution that is isotonic with the blood of the patient to be treated. The saline solution is preferably a NaCl solution. For humans and most mammals, a 0.9 w/w% NaCl solution is isotonic and is preferred as physiological saline solution. However, other pharmaceutically accepted salt or combinations thereof can be chosen to arrive at an isotonic solution. Although not necessary, the composition can comprise additional adjuvants that are commonly known in the art. The composition can be combined with usual care such as anti-biotics and with anti-viral agents that are commonly known in the art.

Attractively, the phenol is in a composition free of lidocaine, i.e. administered without lidocaine in the same composition. It has been found that lidocaine may provide some relieve with regard to local discomfort as a result of the injections, but as a compound for treatment of the envisaged condition, lidocaine is not regarded as an active ingredient, but may cause undesired side effects. Therefore, it is preferred to administer phenol in absence of lidocaine. The composition preferably consists of phenol in physiological saline.

In an attractive embodiment, the phenol is administered in a liquid composition comprising 0.005 - 0.07 w/w%, preferably 0.01 - 0.06 w/w% phenol, most preferably in physiological saline.

In an particularly attractive embodiment, the phenol is injected subcutaneously, i.e. in the soft tissue below the skin with a depth of up to 0.5 - 2.0 cm. By such an injection, the phenol will diffuse fast to the neighbouring locations and exert its antioxidant action. According to the clinical observation, phenol may remain about 24 hours in the body, with an immediate effect of about 7 days. Based on studies done on diabetic patients that inject insulin comprising 0.2 to 1.0 mg per dose phenol as preservative, there is no significant accumulation of phenol in the body by daily use.

A high dose would damage the body, but too low will show no effect. The dose is preferably 0.4 - 3.0 mg phenol once daily, more preferably 0.8 - 2 mg phenol once daily. If the symptoms are mild or decreasing, the dose can be lowered to e.g. every other day or a dose once weekly of e.g. the same amounts. The dose is preferably provided in a liquid volume of 0.3 - 5.0 ml, more preferably of 0.3 - 3.0 ml, even more preferably 0.5 - 2.5 ml, preferably of an isotonic saline solution, more preferably physiological saline, i.e. 0.9 w/w% NaCl. Although not necessary, the composition can comprise not only phenol and saline, but optionally additional adjuvants that are commonly known in the art. If necessary, the composition can comprise a local anaesthetic in order to avoid any pain effect upon injection. As discussed above, the presence of lidocaine is not preferred.

A single dose is intended to relate to the amount of phenol administered subcutaneously, wherein the administration can be divided over a plurality of injections at different locations, as long as these injections are performed together, i.e. one after the other, not more than 10 minutes, preferably not more than 5 minutes, even more preferably not more than 3 minutes being between each injection. The above dose is preferably divided over two to ten subdoses. In particular for obese patients and patient having a high level of oxidative activity, e.g. as a result of an immune disorder are candidates for receiving the dose in 6 to 10 injections. For the majority of patients, four to six subdoses are preferred.

The dose is preferably equally divided over the two to ten subdoses, preferably four to six subdoses, both in volume and phenol concentration, so that the different locations each receive an equal subdose.

For the treatment of dyspnoea, local administration is found to be most effective; particularly preferred is the administration in the neck area, in particular close to the central nerve systems and close to the airways. The neck area is most accessible and practical location to deposit the phenol as close as possible at the location of the dyspnoea.

The locations of injection points are preferably chosen with the objective to coordinate the central autonomic nerve, and regulate both sympathetic and parasympathetic system and to provide the antioxidant signals to the local airway and entry of the lungs at the points before and behind the entry of lungs, and to release tension of the neck muscles to provide better circulation to the brain. The term 'neck area' encompasses the area between the head and the shoulders, limited to above the 8^{th} vertebra.

Preferably, a dose for administration in the neck area is divided over four to ten, more preferably over four to six, even more preferably over four injections. Said subdoses preferably comprise 0.4 - 3.0 mg, more preferably 0.8 - 2.0 mg each. The volume of the subdoses are preferably 0.3 - 0.8 ml each, whereas the phenol concentration in the composition to be administered is preferably 0.06 w/w%.

Preferably the subdoses comprise 4 injections of 0.5 ml comprising 0.06 w/w% phenol. In case of a different number of injections, the preferred volume per injection is 0.5 ml, with a composition comprising 0.06 w/w% phenol. To this end, the different positions in the neck area are preferably chosen from the group, consisting of: the posterior margin of the sternocleidomastoid muscle on the level of the larynx knot at both left and right side of the body; the middle of the upper socket of the sternum, and the depression below the spinous process of the 7^{th} cervical vertebra.

In another attractive embodiment, the phenol is administered intravenously. By such administration, the phenol will diffuse very fast throughout the patient's body and exerts its antioxidant action. As for subcutaneous administration, phenols may remain about 24 hours in the body, with an immediate effect of about 7 - 14 days.

The dose of phenol to be administered intravenously is preferably 0.02 - 0.1 mg per kg body weight per day, more preferably 0.03 - 0.07 mg per kg body weight per day. The stronger the dyspnoea or the underlying disease manifests, the higher the dose.

The dose is preferably administered for numerous consecutive periods, until recovery of the patient is observed. Such period is preferably 3 to 8 consecutive days, weeks, alternating weeks. However, if the patient is recovering, the dose can be lowered, either in amount, or in administration interval, or both. For example, a dose can be administered each second day or each week or alternating week or month, when recovery is observed.

Therefore, in a preferred embodiment, as from the day following the last day of the said dose administration, a second dose of 0.005 - 0.2 mg per kg body weight per day phenol is administered for 1- 5 days. Such additional treatment can also comprise one or more daily subcutaneous injections, in particular in the neck area as described above. To this end, the total dose of said subcutaneous injections are preferably 0.4 - 1.5 mg per day, where the injections preferably are divided multiple injections of 0.8 - 1.5 mg in 0.3 - 0.8 ml each, in particular at four positions in the neck, that are preferably chosen from the group, consisting of the middle of the anterior and posterior margins of the sternocleidomastoid muscle on the level of the larynx knot at both left and right side of the body; the middle of the upper socket of the sternum, and the depression below the spinous process of the 7^{th} cervical vertebra.

The dose can be administered intravenously as a single or multiple bolus injections, but is preferably administered by infusion, in particular by drip infusion, where the phenol concentration is preferably 0.005 - 0.07 w/w%. The infusion is preferably performed for 3 minutes to two hours. In particular when the patient is recovering, the infusion time can be short, e.g. 3 to 30 minutes.

The invention also describes an infusion composition, in particular for drop infusion, comprising 0.005 - 0.07 w/w%, preferably 0.01 - 0.06 w/w% phenol in physiological saline. The infusion composition is preferably packaged as such, e.g. in a plastic bag, preferably comprising outlets designed for infusion tubing to be connected thereto, and e.g. suspending means to be suspended from a frame, designed for suspending infusion bags.

### Example 1

### Virus induced pneumonia

Subject 1 was a male, 60 years old, weight 85 kg, pre-condition asthma. Probably virus induced pneumonia. Pre-treatment history:
Suffering from dry throat, slight headache, started coughing after three days, next day short breath, dyspnoea. thought it was a common cold and asthma. In the evening of fourth day fever. Self-treatment with Ventolin and paracetamol. Fever reduced slightly, but still coughing. Sixth day. Still fever. Measured temperature 37.8°C. Short of breath, and pain in the chest. Felt very tired. Insomnia. Eighth day: Cough with lot of mucus. Patient thinks that the problem is serious, and contacts GP. Diagnosis virus induced pneumonia. Temperature 38.1°C.

Treatment started for 9 consecutive days by four subcutaneous injections of a 0.06 w/w% phenol in physiological saline daily administered in: 1 and 2) the posterior margin of the sternocleidomastoid muscle on the level of the larynx knot at both left and right side of the body, each about 1 cm deep; 3) the middle of the upper socket of the sternum about 0.5 cm deep, and 4) the depression below the spinous process of the 7^{th} cervical vertebra, about 0.5 cm deep. First day of treatment is set as 'Day 1'.

### Observations:

Day 1: Temperature 38.1°C.
Day 2: Temperature 37.6°C. A lot of transparent slime. Chest pain. Limited feeling of short breath and asthma. Tired feeling.
Day 3: Temperature 37.4°C. Less short breath. Tired. Feels some pain at injection point during treatment. Slight headache after injection.
Day 4: Temperature 37.4°C, coughing, tired but less tired than before. Short breath much better. No chest pain by coughing.
Day 5. Temperature 37.4°C. coughing, less tired. Short breath slightly better.
Day 6: Temperature 37.0°C. Slept reasonable. Still coughing, but less. Says feels good. No more short breath.
Day 7: No fever. Feels good. Feeling energy back. Still light coughing. No pain. Breathing normal.
Day 8: No fever. Almost no cough.
Day 9: As Day 8. Last day of injections.

Analysis: Patient experienced a slight headache, no other side effect after injection. Patient stopped with Ventolin and paracetamol on treatment Day 2.

### Example 2

### Early stage CoViD-19

Subject 2 male, 29 years, suffering early stage CoViD-19. Former professional sportsman (soccer), doesn't smoke, no alcohol, weight 75 kg. Pre-treatment history. Patient felt good in the morning. Around noon started irritation of the throat, problems sleeping. Next day felt tired, started coughing, in the afternoon feeling feverish, problems sleeping. Next Day: Coughing increased, strong pain in the chest when coughing, dyspnoea. Feels to have fever. Sleeping problems. Next day pain in the chest increases. Shortness of breath increases. Came to GP for treatment. Fever 38.4°C.

Treatment for four consecutive days of four subcutaneous injections as described for patient 1. First day of treatment is set as 'Day 1'.

### Observations:

Day 1: Temperature 38.4°C.
Day 2: After treatment the patient felt a slight headache which remained for about four hours. Slept reasonable, only interrupted by coughing. Coughing less, almost no more pain in the breast when coughing. Still feels weak. Temperature 37.2°C.
Day 3: Coughing lessened further, no more pain in the chest when coughing. Temperature 36.8°C. Patient feels normal, not weak anymore. Slept well.
Day 4: Temperature normal. Slight cough.
Day 5: Temperature normal. the slight cough remained for 5 more days and then is fully gone. No further injections administered.

Analysis: No headache except after first treatment. Injection location slight pain at injection. No other side effects noted.

### Example 3

Treatment of critical stage Covid-19, treatment of diffuse intravascular coagulation

Patient cured with the said injection. Clinical observations, 69+ years, obese, fever and coughing 10 days, fever fluctuating for 7 days. R/R 41, SpO₂ 91%, intubation, D-dimer test was 1.1 µg/mL infusion (sodium glucose). Treated with concentration phenol 0.01% in 60 ml, drip infusion 20 minutes, once daily. After recovery to no fever, treatment was continued by drip infusion 15 minutes, once daily with 30 ml infusion medium with a phenol concentration of 0.01%. Treatment continued till 3 days normal body temperature, R/R 20, SPO₂ 97%.

Recovering process: improvement of R/R and SpO₂ measured 6 hours after start treatment, day 6 normal temperature, R/R, SpO₂. Day 6 lowered dose to 0.01% concentration in 30ml. Stopped treatment after treatment day 9. Patient recovered, see table 1.

### Example 4

MRSA lung infection post operation prostate cancer.

Male, 72 years, 88 kg, dyspnoea, cough, chest pain and fever, under urine catheter. Has used different antibiotic before and after operation prostate cancer and by lung infection. Fever did not reduce. MRSA cultivation positive.

Treatment 0.02% phenol 30ml infusion in about 15 minutes and once per day for continuously 3 days and after fever reduce from 38.0 to 37.4°C. Phenol reduce to 0.02% 15 ml. duration 10 minutes for 5 more days. The temperature was stable under 37°C. Day 18th the X-lung showed that pneumonia was almost recovered.

### Example 5

### Flu pneumonia.

Female, 84 years, 72 kg, fever, chest pain, headache, and cough grey mucus for two weeks, dyspnoea. No response to antibiotics. Patient developed diarrhoea and became weaker and weaker. Heartbeat 120/minute and irregular. R/R 29/minute. Temperature between 37.5 - 38.6°C. X-lungs right middle and under lobes / left under lobe show pulmonary infiltrates. Treatment 0.02% phenol 30 ml intravenous and duration 10 minutes once per day for continuously 5 days. Day 5: breaths quietly and heartbeat about 90 per minute. Fever 36.8 - 37.5°C. Additional treatment four local neck plus one between 3 - 4 thorax vertebrae subcutaneous injection for another 6 more days. Each location 0.5 ml 0.06% subcutaneous. Patient was physical recovered with some cough. X-lung infiltrate on day 14 much improved

**Table 1: Example 3 Patient treatment and recovery**

| | | | day1 | day2 | day3 | day4 | day 5 | day6 | day7 | day8 | day9 |
|---|---|---|---|---|---|---|---|---|---|---|---|
| sex | male | | | | | | | | | | |
| age | 69 | | | | | | | | | | |
| weight | 92 | | | | | | | | | | |
| BMI | 31.5 | | | | | | | | | | |
| diagnosis | pre treatment | 10 days fever. Fever up to 38.9. Alternating | | | | | | | | | |
| | | risk of disseminated intravascular coagulation | | | | | | | | | |
| | | Covid19 critical phase | | | | | | | | | |
| | | Blue lips | | | | | | | | | |
| | | R/R 41 | 41 | 32 | 27 | 23 | 22 | 20 | 20 | 20 | 18 |
| | | SpO₂ 91% | 91% | 93% | 94% | 96% | 96% | 97% | 97% | 97% | 97% |
| | | body temperature 37.6°C | 37.6°C | 38.6°C | 38.5°C | 38.0°C | 37.4°C | 37.0°C | 36.8°C | 36.9°C | 36.8°C |
| | | lung infiltrate | | | | | | | | | |
| therapy | intubation | | y | y | n | n | n | n | n | n | n |
| | infusion (0.9%NaCl) | | y | y | y | y | y | y | y | y | y |
| | azitromycine | | n | n | n | n | n | n | n | n | n |
| treatment | infusion | phenol 0.02% (in 30ml), drip 15 minutes | y | y | y | y | y | | | | |
| | | phenol 0.02% (in 15ml), drip 10 minutes | | | | | | y | y | y | y |
| | | | | | | | | | | | |
| day 6 evaluation | | patient breaths normally, still stress, coughing, feels good, day 6 started walking (with infusion) | | | | | | | | | |
| day 9 evaluation | | patients three days normal body temperature. | | | | | | | | | |

## Claims

1. Phenol for use in the treatment of dyspnoea in a patient, the dyspnoea being associated with Acute Respiratory Distress Syndrome (ARDS) and being caused by a coronavirus, wherein the phenol is administered parenterally, chosen from subcutaneous injection and intravenous administration.

2. Phenol for use of claim 1, wherein the patient is a human.

3. Phenol for use of any of the preceding claims, wherein the dyspnoea is caused by CoViD-19.

4. Phenol for use of any of the preceding claims, wherein the phenol is in a composition free of lidocaine.

5. Phenol for use of any of the preceding claims, wherein the phenol is in a physiological saline composition.

6. Phenol for use of any of the preceding claims, wherein the phenol is administered in a liquid composition comprising 0.005 - 0.07 w/w% phenol, preferably 0.01 - 0.06 w/w% phenol.

7. Phenol for use of any of the preceding claims, wherein the phenol is administered by subcutaneous injection.

8. Phenol for use of claim 7, wherein the dose is:
- 0.4 - 3.0 mg once daily, preferably 0.8 - 2.0 mg once daily, or
- 0.4 - 3.0 mg once weekly, preferably 0.8 - 2.0 mg once weekly, and/or
- is in 0.3 - 3.0 ml physiological saline, preferably 0.3 - 0.8 ml physiological saline.

9. Phenol for use according to claim 7 or 8, wherein the dose is divided over 2 to 10 subdoses, preferably 4 to 6 subdoses,
- the dose preferably being equally divided over the two to ten subdoses;
- the volume of the subdoses preferably being 0.3 - 0.8 ml each.

10. Phenol for use of any of the claims 7 - 9, wherein the phenol is administered in the neck area, the dose preferably being divided over two to ten subdoses, to be administered on different positions in the neck area, the different positions in the neck area preferably being chosen form the group, consisting of: the posterior margin of the sternocleidomastoid muscle on the level of the larynx knot at both left and right side of the body; the middle of the upper socket of the sternum, and the depression below the spinous process of the 7^{th} cervical vertebra.

11. Phenol for use of any of the preceding claims, wherein the phenol is administered intravenously.

12. Phenol for use of claim 11, wherein:
- the dose is 0.02 - 0.1 mg per kg body weight per day, and/or
- the dose is administered daily for 3 - 8 consecutive days, wherein preferably as from the day following the last day of the said dose administration a second dose of 0.005 - 0.3, in particular 0.005 - 0.2 mg per kg body weight per day phenol is administered for 1 - 5 days.

13. Phenol for use of claim 11 or 12, wherein the dose is administered by infusion, in particular by drip infusion, preferably administered for 10 minutes to 2 hours.

## Patentansprüche

1. Phenol zur Verwendung bei der Behandlung von Dyspnoe bei einem Patienten, wobei die Dyspnoe mit dem akuten Atemnotsyndrom (ARDS) in Verbindung steht und durch ein Coronavirus verursacht wird, wobei das Phenol parenteral verabreicht wird, ausgewählt aus subkutaner Injektion und intravenöser Verabreichung.

2. Phenol zur Verwendung nach Anspruch 1, wobei der Patient ein Mensch ist.

3. Phenol zur Verwendung gemäß einem der vorstehenden Ansprüche, wobei die Dyspnoe durch CoViD-19 verursacht wird.

4. Phenol zur Verwendung gemäß einem der vorstehenden Ansprüche, wobei das Phenol in einer Zusammensetzung ohne Lidocain vorliegt.

5. Phenol zur Verwendung gemäß einem der vorstehenden Ansprüche, wobei das Phenol in einer physiologischen Kochsalzlösung vorliegt.

6. Phenol zur Verwendung gemäß einem der vorstehenden Ansprüche, wobei das Phenol in einer flüssigen Zusammensetzung verabreicht wird, die 0,005 bis 0,07 Gew.-% Phenol, vorzugsweise 0,01 bis 0,06 Gew.-% Phenol, umfasst.

7. Phenol zur Verwendung gemäß einem der vorstehenden Ansprüche, wobei das Phenol durch subkutane Injektion verabreicht wird.

8. Phenol zur Verwendung gemäß Anspruch 7, wobei die Dosis:
- 0,4 bis 3,0 mg einmal täglich, vorzugsweise 0,8 bis 2,0 mg einmal täglich ist, oder
- 0,4 bis 3,0 mg einmal wöchentlich, vorzugsweise 0,8 bis 2,0 mg einmal wöchentlich ist, und/oder
- in 0,3 bis 3,0 ml physiologischer Kochsalzlösung, vorzugsweise 0,3 bis 0,8 ml physiologischer Kochsalzlösung ist.

9. Phenol zur Verwendung gemäß Anspruch 7 oder 8, wobei die Dosis auf 2 bis 10 Teildosen, vorzugsweise 4 bis 6 Teildosen, aufgeteilt ist, wobei
- die Dosis vorzugsweise gleichmäßig auf die zwei bis zehn Teildosen aufgeteilt ist;
- das Volumen der Unterdosen vorzugsweise jeweils 0,3 bis 0,8 ml beträgt.

10. Phenol zur Verwendung gemäß einem der Ansprüche 7 bis 9, wobei das Phenol im Halsbereich verabreicht wird, wobei die Dosis vorzugsweise auf zwei bis zehn Unterdosen aufgeteilt wird, die an verschiedenen Stellen im Halsbereich verabreicht werden, wobei die verschiedenen Stellen im Halsbereich vorzugsweise aus der Gruppe ausgewählt werden, bestehend aus: dem hinteren Rand des M. sternocleidomastoideus auf Höhe des Kehlkopfknötchens auf der linken und rechten Seite des Körpers; der Mitte der oberen Aussparung des Brustbeins und der Vertiefung unterhalb des Dornfortsatzes des 7. Halswirbels.

11. Phenol zur Verwendung gemäß einem der vorstehenden Ansprüche, wobei das Phenol intravenös verabreicht wird.

12. Phenol zur Verwendung gemäß Anspruch 11, wobei:
- die Dosis 0,02 - 0,1 mg pro kg Körpergewicht pro Tag beträgt und/oder
- die Dosis täglich über 3 bis 8 aufeinanderfolgende Tage verabreicht wird, wobei vorzugsweise ab dem Tag nach dem letzten Tag der genannten Dosisverabreichung eine zweite Dosis von 0,005 bis 0,3, insbesondere 0,005 bis 0,2 mg pro kg Körpergewicht pro Tag Phenol über 1 bis 5 Tage verabreicht wird.

13. Phenol zur Verwendung nach Anspruch 11 oder 12, wobei die Dosis durch Infusion, insbesondere durch Tropfinfusion, vorzugsweise über einen Zeitraum von 10 Minuten bis 2 Stunden verabreicht wird.

## Revendications

1. Phénol pour l'utilisation dans le traitement de la dyspnée chez un patient, la dyspnée étant associée au syndrome de détresse respiratoire aiguë (SDRA) et étant induite par voie coronavirus, dans lequel le phénol est administré par voie parentérale, choisie parmi l'injection sous-cutanée et l'administration intraveineuse.

2. Phénol pour l'utilisation selon la revendication 1, dans lequel le patient est un être humain.

3. Phénol pour l'utilisation dans l'une quelconque des revendications précédentes, dans lequel la dyspnée est causée par le CoViD-19.

4. Phénol v dans l'une quelconque des revendications précédentes, dans lequel le phénol se trouve dans une composition exempte de lidocaïne.

5. Phénol pour l'utilisation dans l'une quelconque des revendications précédentes, dans lequel le phénol se trouve dans une composition saline physiologique.

6. Phénol pour l'utilisation selon l'une quelconque des revendications précédentes, dans lequel le phénol est administré dans une composition liquide comprenant 0,005 à 0,07 % en poids de phénol, de préférence 0,01 à 0,06 % en poids de phénol.

7. Phénol pour l'utilisation selon l'une quelconque des revendications précédentes, dans lequel le phénol est administré par injection sous-cutanée.

8. Phénol pour l'utilisation selon la revendication 7, dans lequel la dose est :
- de 0,4 à 3,0 mg une fois par jour, de préférence de 0,8 à 2,0 mg une fois par jour, ou
- de 0,4 à 3,0 mg une fois par semaine, de préférence de 0,8 à 2,0 mg une fois par semaine, et/ou
- dans 0,3 à 3,0 ml de solution saline physiologique, de préférence 0,3 à 0,8 ml de solution saline physiologique.

9. Phénol pour l'utilisation selon la revendication 7 ou 8, dans lequel la dose est divisée en 2 à 10 sous-doses, de préférence 4 à 6 sous-doses,
- la dose étant de préférence divisée de manière égale entre les deux à dix sous-doses ;
- le volume des sous-doses étant de préférence de 0,3 à 0,8 ml chacune.

10. Phénol pour l'utilisation selon l'une quelconque des revendications 7 à 9, dans lequel le phénol est administré dans la région du cou, la dose étant de préférence répartie en deux à dix sous-doses, à administrer à différents endroits de la région du cou, les différents endroits de la région du cou étant de préférence choisis parmi le groupe constitué par : le bord postérieur du muscle sterno-cléido-mastoïdien au niveau du nœud laryngé des côtés gauche et droit du corps ; le milieu de la cavité supérieure du sternum, et la dépression sous l'apophyse épineuse de la 7^{e} vertèbre cervicale.

11. Phénol pour l'utilisation selon l'une quelconque des revendications précédentes, dans lequel le phénol est administré par voie intraveineuse.

12. Phénol pour l'utilisation selon la revendication 11, dans lequel :
- la dose est de 0,02 à 0,1 mg par kg de poids corporel par jour, et/ou
- la dose est administrée quotidiennement pendant 3 à 8 jours consécutifs, de préférence à partir du lendemain du dernier jour de ladite administration de dose, une deuxième dose de 0,005 à 0,3, en particulier de 0,005 à 0,2 mg par kg de poids corporel par jour de phénol est administrée pendant 1 à 5 jours.

13. Phénol pour l'utilisation selon la revendication 11 ou 12, dans lequel la dose est administrée par perfusion, en particulier par perfusion goutte à goutte, de préférence administrée pendant 10 minutes à 2 heures.
